# EUROPÄISCHE PATENTSCHRIFT

(11) **EP 0 587 030 B1**
(45) Veröffentlichungstag und Bekanntmachung des Hinweises auf die Patenterteilung: **02.12.1998**
(21) Anmeldenummer: 93113950.5
(22) Anmeldetag: 01.09.1993
(51) Int. Cl.: C09B 62/503, D06P 3/66, D06P 3/02

(54) **Reaktivfarbstoffe auf Formazanbasis sowie Hydrazone**
Reactive dyes based on formazane and hydrazones
Colorants réactifs à base de formazane et des hydrazones

(30) Priorität: 09.09.1992 DE 4230095
(43) Veröffentlichungstag der Anmeldung: 16.03.1994
(73) Patentinhaber: BASF Aktiengesellschaft, 67063 Ludwigshafen (DE)
(72) Erfinder: Marschner, Claus, Dr., D-6720 Speyer (DE); Patsch, Manfred, Dr., D-6706 Wachenheim (DE)

(56) Entgegenhaltungen:
- EP-A- 0 028 787
- EP-A- 0 028 788
- EP-A- 0 135 040
- EP-A- 0 183 054
- DE-A- 3 326 638
- US-A- 4 607 098

## Beschreibung

Die vorliegende Erfindung betrifft neue Formazanfarbstoffe der Formel I 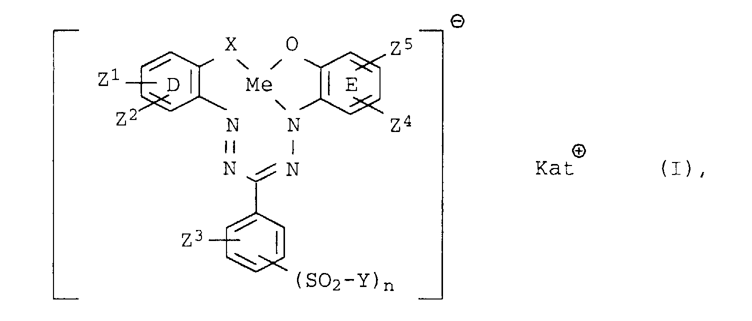 in der
- n: 1 oder 2,
- Kat^{⊕}: das Äquivalent eines Kations,
- Me: Kupfer oder Nickel,
- X: einen Rest der Formel CO-O oder SO₂-O
- Y: Vinyl oder einen Rest der Formel C₂H₄-Q, worin Q für eine unter alkalischen Reaktionsbedingungen abspaltbare Gruppe steht,
- Z¹: Wasserstoff oder Hydroxysulfonyl,
- Z²: Wasserstoff, C₁-C₄-Alkanoylamino, Halogen oder Nitro,
- Z³: Wasserstoff, Halogen, C₁-C₄-Alkyl, C₁-C₄-Alkoxy, C₁-C₄-Alkylsulfonyl, Nitro oder Hydroxysulfonyl und
- Z⁴ und Z⁵: unabhängig voneinander jeweils Hydroxysulfonyl, Halogen, Nitro, Carboxyl, Ureido, gegebenenfalls substituiertes C₁-C₄-Mono- oder Dialkylureido, gegebenenfalls substituiertes Phenylureido, gegebenenfalls substituiertes Phenyl, gegebenenfalls substituiertes C₁-C₄-Alkanoylamino, gegebenenfalls substituiertes Benzoylamino, C₁-C₄-Alkoxycarbonylamino, gegebenenfalls substituiertes Mono- oder Dialkylcarbamoyl, gegebenenfalls substituiertes Mono- oder Dialkylsulfamoyl, einen Rest der Formel SO₂-Y oder einer der beiden Reste Z⁴ und Z⁵ auch Wasserstoff bedeuten und die Ringe D und E benzoanelliert sein können,
deren Verwendung zum Färben oder Bedrucken von Hydroxygruppen oder Stickstoffatome aufweisenden organischen Substraten sowie neue Hydrazone.

Metallkomplex-Formazanfarbstoffe, die reaktive Gruppen aufweisen, sind an sich bekannt und z.B. in der EP-A-28 788 beschrieben. Die Farbstoffe des Standes der Technik weisen jedoch häufig Mängel in ihren anwendungstechnischen Eigenschaften auf.

Aufgabe der vorliegenden Erfindung war es daher, neue Reaktivfarbstoffe auf Formazanbasis bereitzustellen, die sich durch ein vorteilhaftes anwendungstechnisches Eigenschaftsprofil auszeichnen.

Demgemäß wurden die eingangs näher bezeichneten Formazanfarbstoffe der Formel I gefunden.

Alle in den obengenannten Formeln auftretenden Alkylgruppen können sowohl geradkettig als auch verzweigt sein.

Wenn in der obengenannten Formel I substituierte Alkylgruppen auftreten, so können als Substituenten z.B. Hydroxysulfonyl, Carboxyl, Hydroxy oder Sulfato in Betracht kommen. In diesem Fall weisen sie in der Regel 1 oder 2 Substituenten auf.

Wenn in der obengenannten Formel I substituierte Phenylgruppen auftreten, so können als Substituenten z.B. C₁-C₄-Alkyl, C₁-C₄-Alkoxy, Halogen, Nitro, C₁-C₄-Alkanoyl,C₁-C₄-Alkanoylamino, Benzoylamino oder Hydroxysulfonyl in Betracht kommen. In diesem Fall weisen sie in der Regel 1 bis 3 Substituenten auf.

Kat^{⊕} stellt des Äquivalent eines Kations dar. Es stellt entweder ein Proton dar oder leitet sich von Metall- oder Ammoniumionen ab. Metallionen sind insbesondere die Lithium-, Natrium- oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-, Dialkyl-, Trialkyl- Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch Sauerstoffatome in Etherfunktion unterbrochen sein kann.

Besonders als Kationen hervorzuheben sind Protonen oder Lithium-, Natrium- oder Kaliumionen.

Der Rest Q steht für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, gegebenenfalls substituiertes Phenylsulfonyloxy, C₁-C₄-Alkanoyloxy, C₁-C₄-Dialkylamino oder ein Rest der Formel 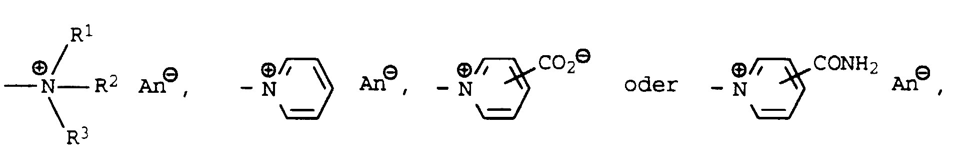 wobei R¹, R² und R³ gleich oder verschieden sind und unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommmen.

Reste Z², Z³, Z⁴ und Z⁵ sind z.B. Fluor, Chlor oder Brom.

Reste Z⁴ und Z⁵ sind weiterhin z.B. N-Methylureido, N-Ethylureido, N-Propylureido, N-Isopropylureido, N-Butylureido, N-(2-Hydroxysulfonylethyl)ureido, N-(2-Sulfatoethyl)ureido, N,N-Dimethylureido, N-Methyl-N-(2-sulfatoethyl)ureido, Phenylureido, Phenyl, 2-, 3- oder 4-Methylphenyl, 2-, 3- oder 4-Methoxyphenyl, 2-, 3- oder 4-Chlorphenyl, 2-, 3- oder 4-Hydroxysulfonylphenyl, Formylamino, Acetylamino, Propionylamino, Butyrylamino, Isobutyrylamino, 3-Hydroxysulfonylpropionylamino, 3-Carboxypropionylamino, Benzoylamino, 2-, 3- oder 4-Hydroxysulfonylbenzoylamino, 2-, 3- oder 4-Chlorbenzoylamino, 2-, 3- oder 4-Methylbenzoylamino, 2-, 3- oder 4-Carboxylbenzoylamino, Methoxycarbonylamino, Ethoxycarbonylamino, Propoxycarbonylamino, Isopropoxycarbonylamino, Butoxycarbonylamino, Carbamoyl, Mono- oder Dimethylcarbamoyl, Mono- oder Diethylcarbamoyl, Mono- oder Dipropylcarbamoyl, Mono- oder Diisopropylcarbamoyl, Mono- oder Dibutylcarbamoyl, N-Methyl-N-ethylcarbamoyl, Mono-(2-hydroxysulfonylethyl)carbamoyl, Sulfamoyl, Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, N-Methyl-N-ethylsulfamoyl, N-Methyl-N-(2-hydroxysulfonylethyl)sulfamoyl, 2-Sulfatoethylsulfonyl, 2-Chlorethylsulfonyl oder 2-Thiosulfatoethylsulfonyl.

Reste Z² sind weiterhin z.B. Formylamino, Acetylamino, Propionylamino, Butyrylamino oder Isobutyrylamino.

Reste Z³ sind weiterhin z.B. Methyl, Ethyl, Propyl, Isopropyl, Butyl, Isobutyl, sec-Butyl, Methoxy, Ethoxy, Propoxy, Isopropoxy, Butoxy, Isobutoxy, sec-Butoxy, Methylsulfonyl, Ethylsulfonyl, Propylsulfonyl, Isopropylsulfonyl oder Butylsulfonyl.

Bevorzugt sind Formazanfarbstoffe der Formel I, in der Me Kupfer bedeutet.

Weiterhin bevorzugt sind Formazanfarbstoffe der Formel I, in der n 1 bedeutet.

Weiterhin bevorzugt sind Formazanfarbstoffe der Formel I, in der Y Vinyl oder einen Rest der Formel C₂H₄-Q bedeutet, worin Q für Chlor, Sulfato oder Thiosulfato steht, wobei Chlor besonders hervorzuheben ist.

Besonders bevorzugt sind Formazanfarbstoffe, die der Formel Ib 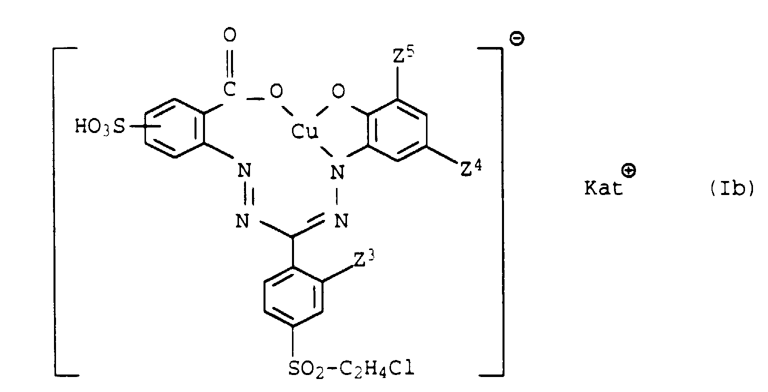 entsprechen, in der
- Z³: Wasserstoff oder Chlor bedeutet und
- Z⁴, Z⁵ und Kat^{⊕}: jeweils die obengenannte Bedeutung besitzen.

Hervorzuheben sind Formazanfarbstoffe der Formel Ic 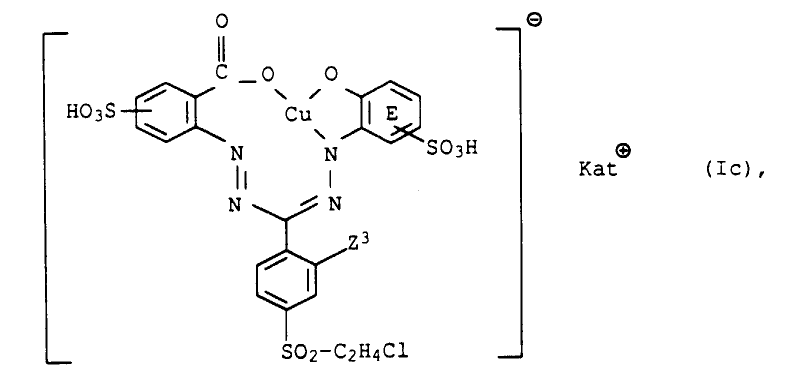 in der
- Z³: Wasserstoff oder Chlor bedeutet und
der Ring E und Kat^{⊕} jeweils die obengenannte Bedeutung besitzen.

Von besonderem Interesse sind Formazanfarbstoffe der Formel Id 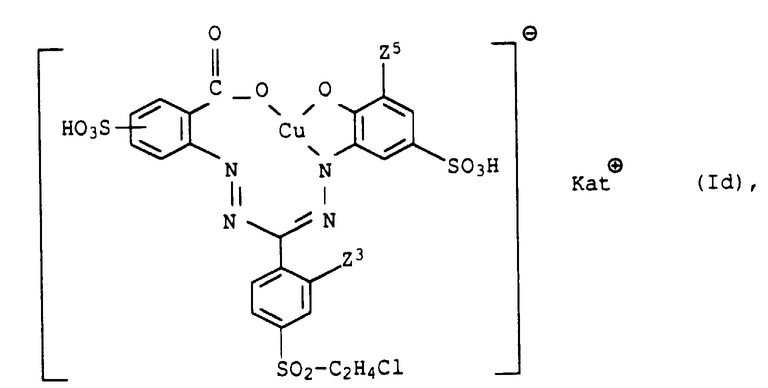 in der
- Z³: Wasserstoff oder Chlor bedeutet und
- Z⁵ und Kat^{⊕}: jeweils die obengenannte Bedeutung besitzen.

Von besonderem Interesse sind weiterhin Formazanfarbstoffe der Formel Ie 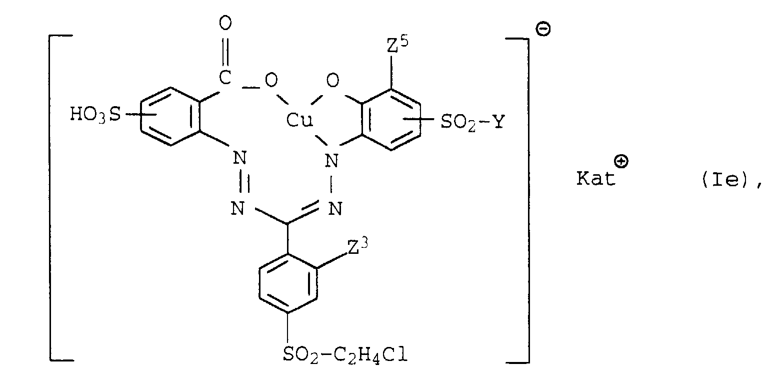 in der
- Z³: Wasserstoff oder Chlor,
- Z⁵: Wasserstoff, Chlor, Brom, Nitro oder Hydroxysulfonyl bedeuten und
- Y und Kat^{⊕}: jeweils die obengenannte Bedeutung besitzen.

Die neuen Formazanfarbstoffe der Formel I können nach an sich bekannten Methoden erhalten werden. Beispielsweise kann man ein Hydrazon der Formel II 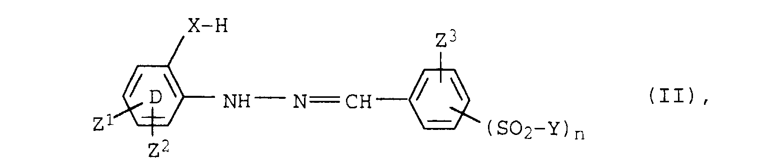 in der n, X, Y, Z¹, Z², Z³ und der Ring D jeweils die obengenannte Bedeutung besitzen, mit einem Diazoniumsalz, das sich von einem Aminophenol der Formel V ableitet 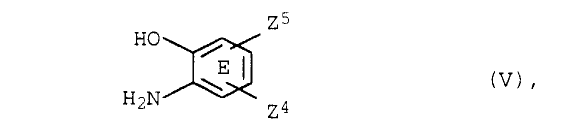 in der der Ring E die obengenannte Bedeutung besitzt, in Gegenwart eines Kupfer- oder Nickelsalzes, z.B. Kupfersulfat oder Nikkelsulfat, umsetzen.

Ein weiterer Gegenstand der vorliegenden Erfindung sind Hydrazone der Formel II 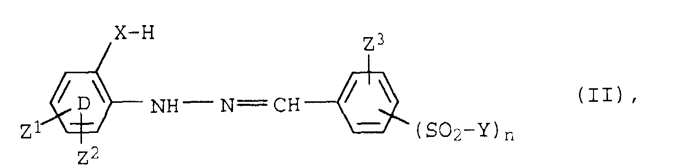 in der
- n: 1 oder 2,
- X: einen Rest der Formel CO-O oder SO₂-O
- Y: Vinyl oder einen Rest der Formel C₂H₄-Q, worin Q für eine unter alkalischen Reaktionsbedingungen abspaltbare Gruppe steht,
- Z¹: Wasserstoff oder Hydroxysulfonyl,
- Z²: Wasserstoff, C₁-C₄-Alkanoylamino, Halogen oder Nitro und
- Z³: Wasserstoff, Halogen, C₁-C₄-Alkyl, C₁-C₄-Alkoxy, C₁-C₄-Alkylsulfonyl, Nitro oder Hydroxysulfonyl bedeuten und
der Ring D benzoanelliert sein kann.

Die neuen Hydrazone der Formel II können vorteilhaft erhalten werden, wenn man z.B. ein Phenylhydrazin der Formel III 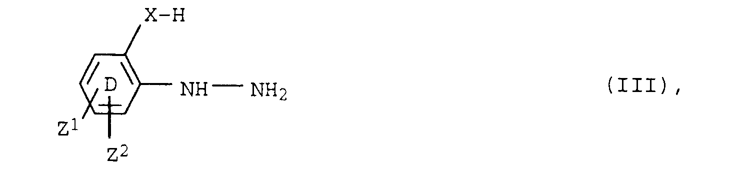 in der X, Z¹, Z² und der Ring D jeweils die obengenannte Bedeutung besitzen, mit einem Benzaldehyd der Formel IV 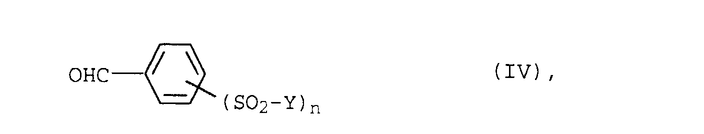 in der n und Y jeweils die obengenannte Bedeutung besitzen, bei einer Temperatur von 30 bis 95°C und einem pH-Wert von 1 bis 5,5 in wäßrigem Milieu kondensiert.

Der pH-Wert kann z.B. unter Verwendung von Salzsäure oder Schwefelsäure eingestellt werden.

Die im erfindungsgemäßen Verfahren zur Anwendung kommenden Phenylhydrazine können nach an sich bekannten Methoden, wie in Houben-Weyl, "Methoden der Organischen Chemie", Band 10/2, Seiten 169 ff, beschrieben, erhalten werden.

Die Benzaldehyde der Formel IV sind in der älteren europäischen Patentanmeldung Nr. 93107419.9 beschrieben.

Die neuen Formazanfarbstoffe der Formel I eignen sich in vorteilhafter Weise zum Färben oder Bedrucken von Hydroxygruppen oder Stickstoffatome aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Wolle oder insbesondere von Baumwolle. Man erhält Ausfärbungen in violetten bis grünen Farbtönen.

Insbesondere auf Substraten auf Basis von Cellulose werden farbstarke Färbungen mit sehr hoher Fixierausbeute erhalten, die eine sehr gute Licht- und Reibechtheit sowie vorzügliche Naßechtheiten, wie Wasch-, Chlorbleich-, Peroxidbleich-, Alkali-, Meerwasser- oder Schweißechtheit, aufweisen.

Die folgenden Beispiele sollen die Erfindung näher erläutern.

### Beispiel 1

41,8 g des Hydrazons der Formel 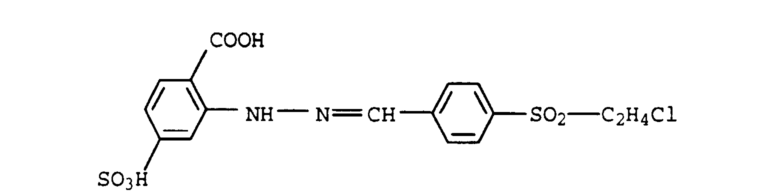 sowie 36,6 g Kupfersulfat-Pentahydrat wurden in 120 ml Wasser suspendiert und durch Zugabe von Natriumcarbonat bei einem pH-Wert von 6,5 bis 7,0 gelost. Zu dieser Lösung ließ man die wäßrige Diazonumsalzlösung, die durch übliche Diazotierung von 56,0 g 2-Aminophenol-4,6-disulfonsäure erhalten wurde, innerhalb von 15 bis 20 Minuten zulaufen und hielt dabei die Temperatur zwischen 10 bis 20°C und den pH-Wert mit Natriumcarbonat bei 6,0 bis 6,5. Man rührte 1 Stunde bis zur Beendigung der Kupplung nach (DC). Zur Überführung des Kupferformazans in die stabile Komplexform wurde anschließend 2 Stunden unter Rückfluß erhitzt.

Nach dem Abkühlen wurde die gebildete Kupferkomplexformazan-Verbindung mit Natriumchlorid ausgefällt, abfiltriert, mit verdünnter wäßriger Natriumchloridlösung gewaschen und bei 50°C unter vermindertem Druck getrocknet.

Man erhielt 103 g kochsalzhaltigen Farbstoff der Formel

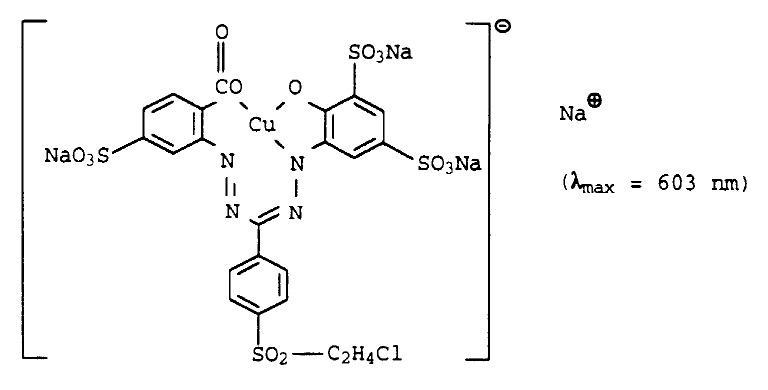

### Beispiel 2

Man verwendete anstelle von 56 g 2-Aminophenol-4,6-disulfonsäure 31 g 6-Acetylamino-2-aminophenol-4-sulfonsäure diazotierte diese auf übliche Weise und verfuhr analog Beispiel 1. Man erhielt 93 g kochsalzhaltigen Farbstoff der Formel

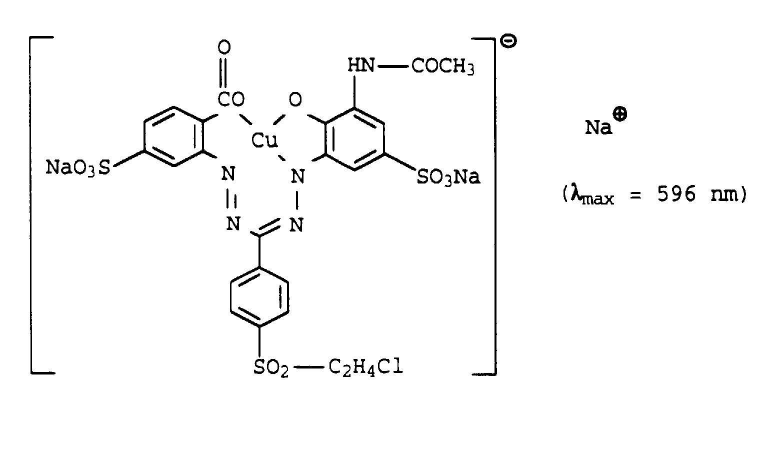

### Beispiel 3

Man verfuhr analog Beispiel 1, setzte jedoch die durch übliche Diazotierung von 33,8 g 6-Acetylamino-2-aminophenol-4-sulfonsäure erhaltene Diazoniumsalzlösung mit 67,9 g des Hydrazons der Formel 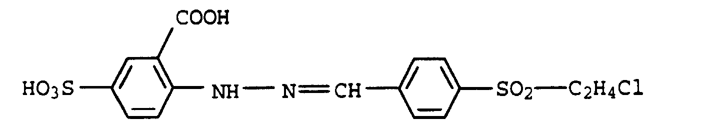 in Gegenwart von 34,0 g Kupfersulfat-Pentahydrat um. Man erhielt 114 g kochsalzhaltigen Farbstoffs der Formel 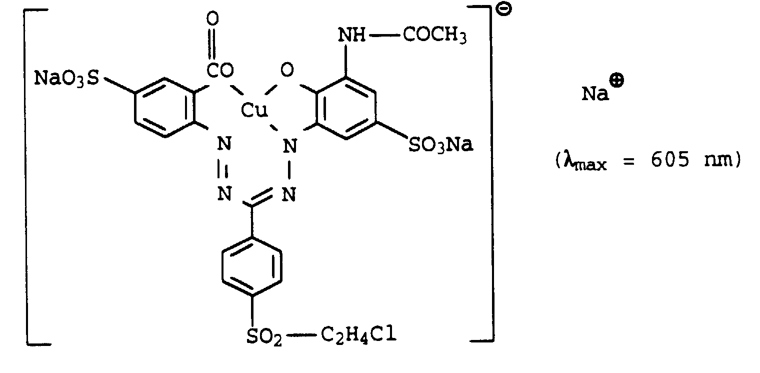 In analoger Weise werden die in der folgenden Tabelle 1 aufgeführten Farbstoffe der Formel 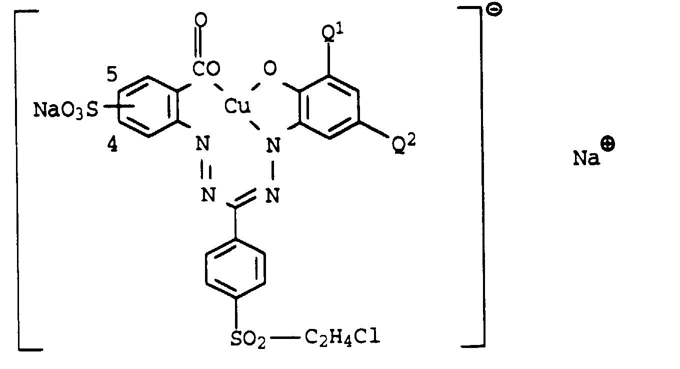 erhalten.

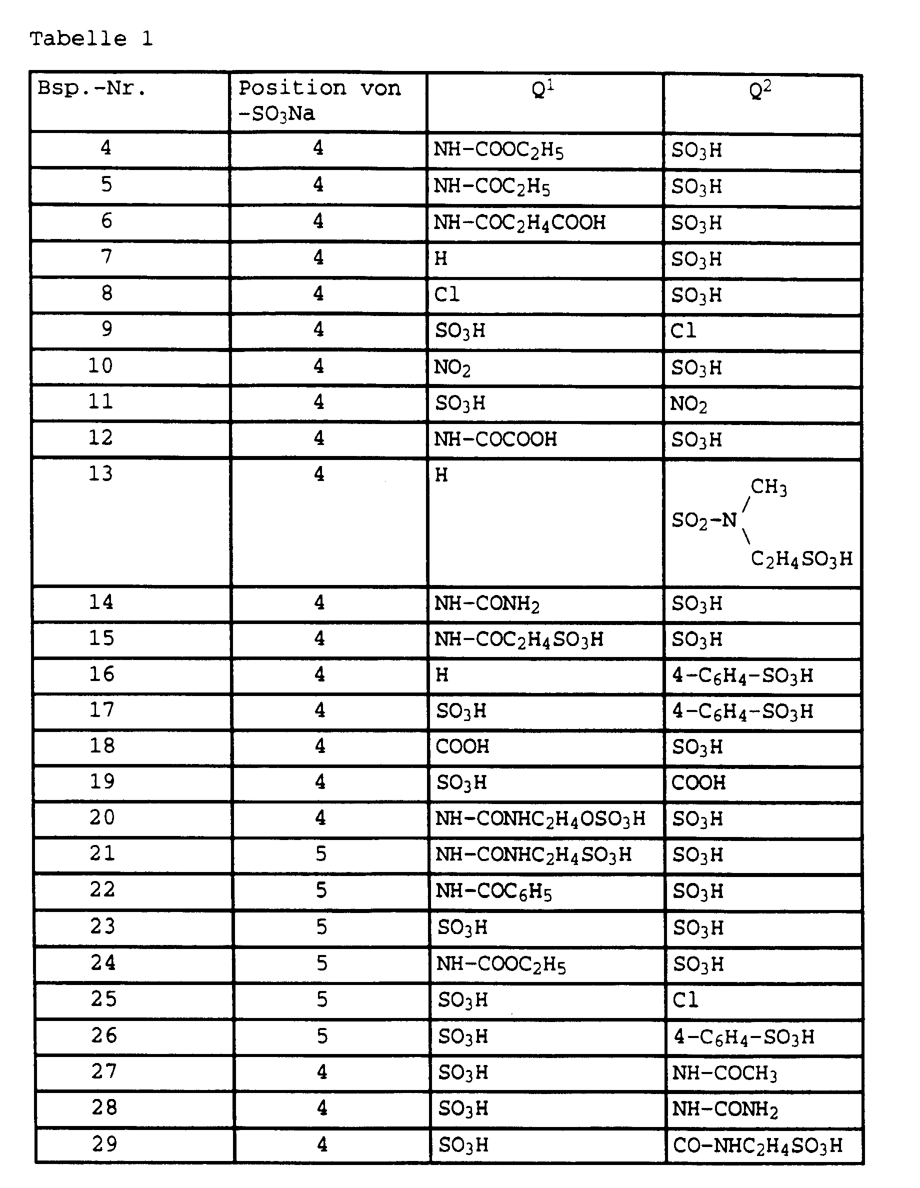

### Beispiel 30

44,4 g des Hydrazons der Formel 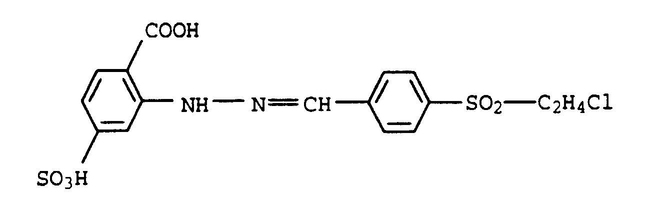 sowie 38,4 g Kupfersulfat-Pentahydrat wurden in 1 l Wasser bei 20 bis 25°C suspendiert und durch Zugabe von 5 gew.-%iger wäßriger Natriumhydrogencarbonatlösung auf einen pH-Wert von 6,5 bis 7,0 eingestellt. Anschließend wurde bei einer Temperatur von 5 bis 15°C eine wäßrige Diazoniumsalzlösung aus 4-(2-Sulfatoethylsulfonyl)-2-aminophenol, die durch übliche Diazotierung in wäßriger Lösung von 29,7 g dieses Aminophenols erhalten wurde, zugegeben, wobei der pH-Wert mit Natriumcarbonat zwischen 5,5 und 6,5 gehalten wurde. Es wurde 12 Stunden bis zur Beendigung der Kupplungsreaktion nachgerührt. Der pH-Wert der Reaktionsmischung wurde mit konzentrierter Salzsäure auf 1 gesenkt und die saure Lösung 2 Stunden nachgerührt. Es wurde mit Natriumhydrogencarbonat bis zu einem pH-Wert von 6,0 bis 6,5 versetzt, die gebildete Kupferkomplex-Formazanverbindung mittels Kochsalz ausgefällt, abfiltriert, mit verdünnter Kochsalzlösung nachgewaschen und unter vermindertem Druck bei 40°C getrocknet. Man erhielt 171 g des kochsalzhaltigen Farbstoffs der Formel 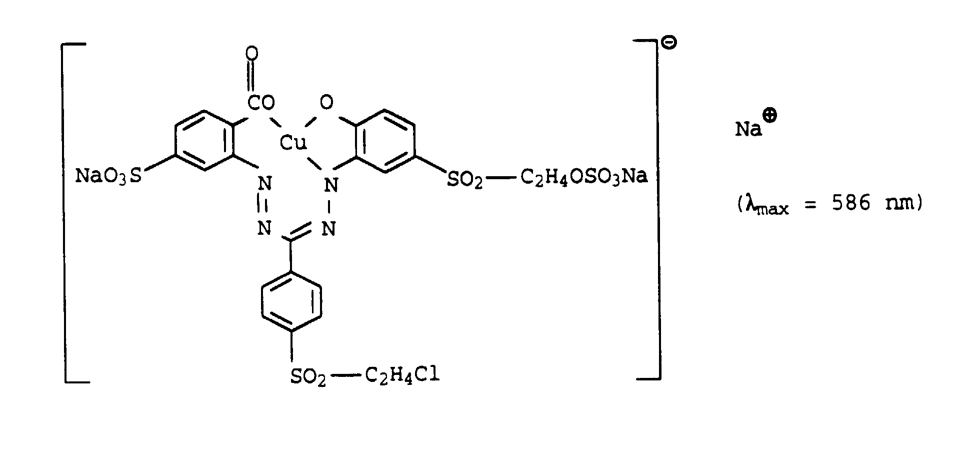 In analoger Weise werden die in der folgenden Tabelle 2 aufgeführten Farbstoffe erhalten.

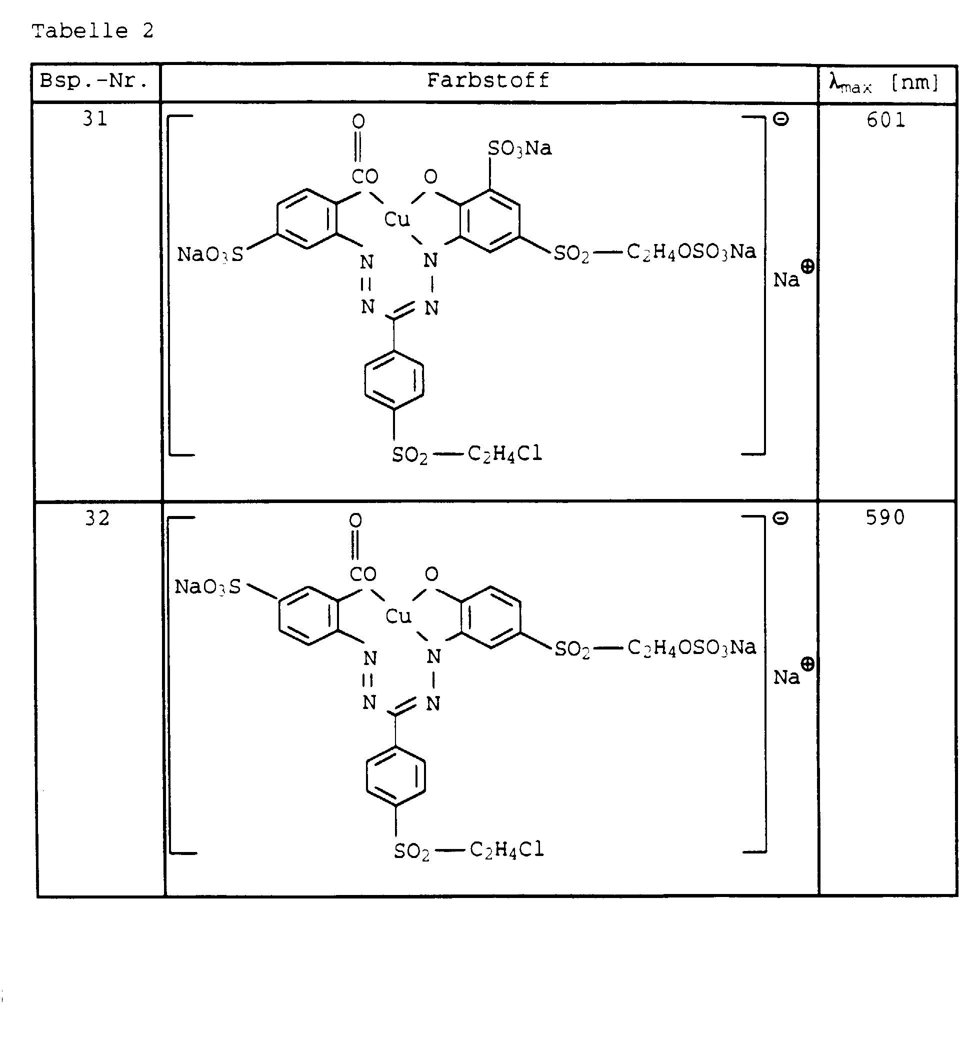

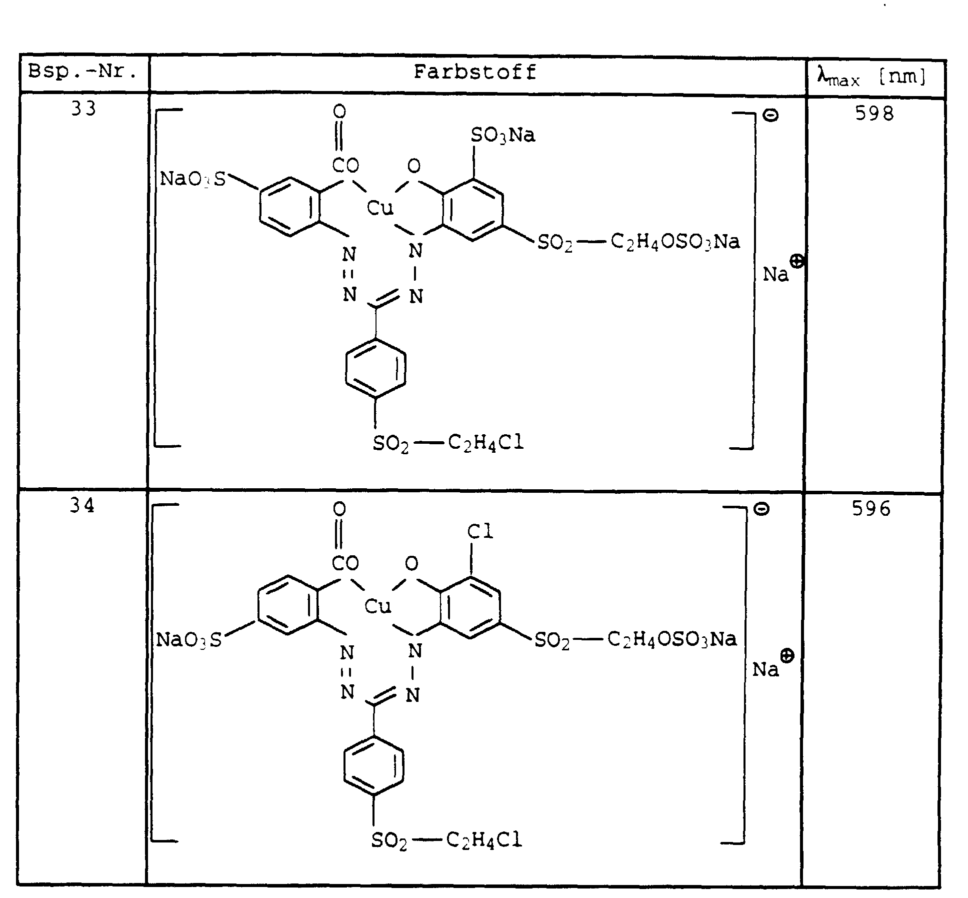

### Beispiel 35

64,4 g des Hydrazons der Formel 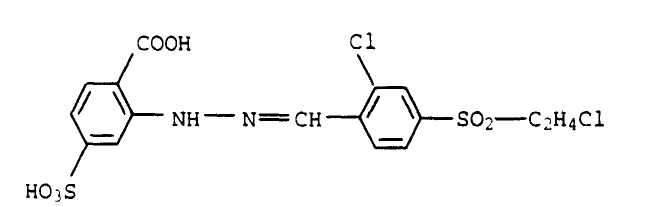 sowie 34,0 g Kupfersulfat-Pentahydrat wurden in 260 ml Wasser suspendiert und durch Zugabe von Natriumcarbonat bei einem pH-Wert von 6,5 bis 7,0 gelöst. Zu dieser Lösung wurde die wäßrige Diazoniumsalzlösung, die durch übliche Diazotierung von 59,5 g 2-Aminophenol-4,6-disulfonsäure erhalten wurde, innerhalb von 15 bis 20 Minuten zugegeben, wobei die Temperatur zwischen 10 bis 25°C und der pH-Wert mit Natriumcarbonat bei 6,0 und 6,5 gehalten wurde. Es wurde 8 Stunden bis zur Beendigung der Kupplung nachgerührt (DC) und anschließend zur Überführung des Kupferformazans in die stabile Komplexform 2 Stunden unter Rückfluß erhitzt. Nach dem Abkühlen wurde die gebildete Kupferkomplex-Formazanverbindung mit Natriumchlorid ausgefällt, abfiltriert, mit verdünnter wäßriger Natriumchloridlösung gewaschen und bei 50°C unter vermindertem Druck getrocknet. Man erhielt 168 g kochsalzhaltigen Farbstoff der Formel

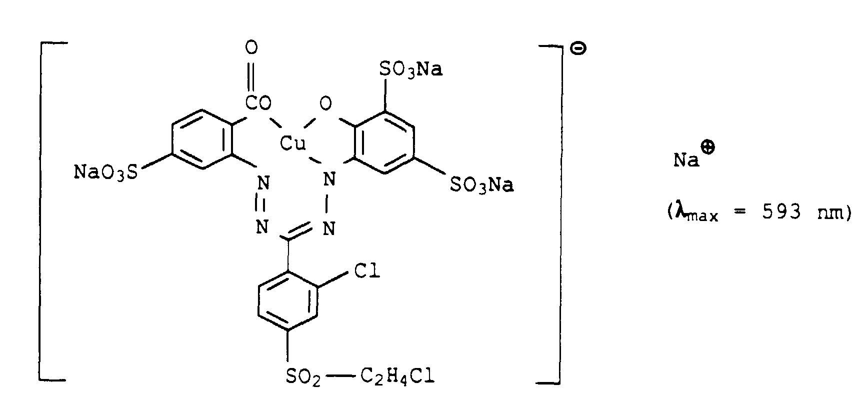

In analoger Weise werden die in der folgenden Tabelle 3 aufgeführten Farbstoffe der Formel

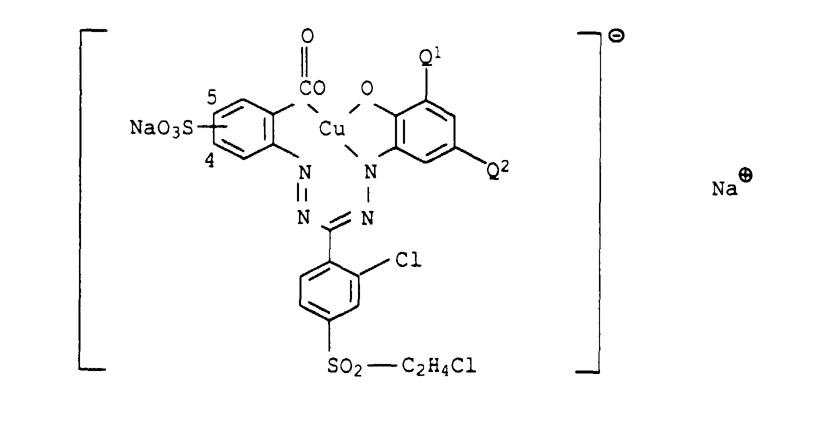

**Tabelle 3**

| Bsp.-Nr. | Position von -SO₃Na | Q¹ | Q² |
|---|---|---|---|
| 36 | 4 | NH-COCH₃ | SO₃H |
| 37 | 4 | H | SO₂-C₂H₄OSO₃H |
| 38 | 4 | SO₃H | SO₂-C₂H₄OSO₃H |
| 39 | 5 | SO₃H | SO₂-C₂H₄OSO₃H |
| 40 | 4 | NH-COOC₂H₅ | SO₃H |
| 41 | 4 | NH-COC₆H₅ | SO₃H |
| 42 | 5 | Cl | SO₃H |
| 43 | 4 | COOH | SO₃H |

### Beispiel 44

Man verfuhr analog Beispiel 2, verwendete jedoch das Hydrazon der Formel 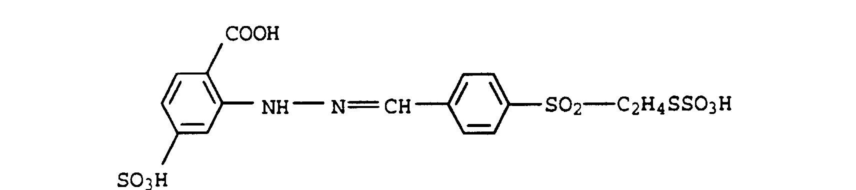 und erhielt 124 g des kochsalzhaltigen Farbstoffs der Formel 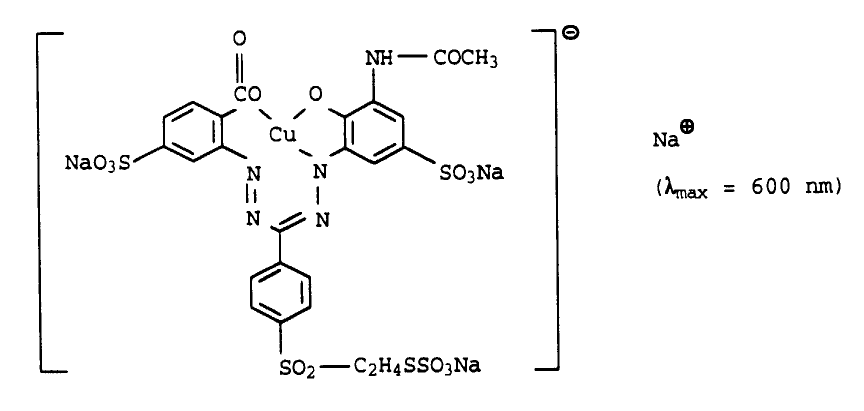 In analoger Weise werden die in der folgenden Tabelle 4 aufgeführten Farbstoffe der Formel 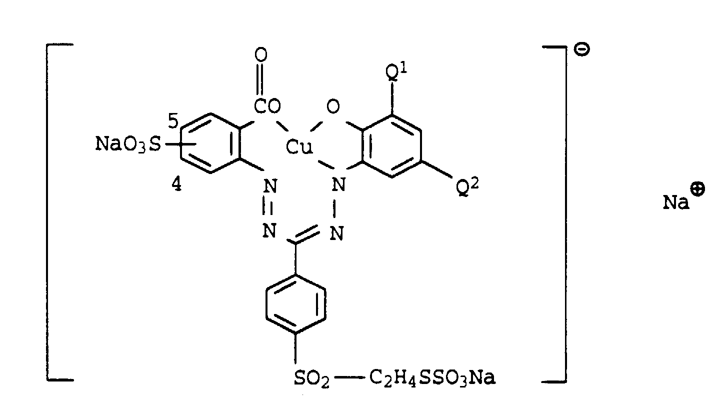 erhalten.

**Tabelle 4**

| Bsp.-Nr. | Position von -SO₃Na | Q¹ | Q² |
|---|---|---|---|
| 45 | 4 | SO₃H | SO₃H |
| 46 | 4 | NH-COOC₂H₅ | SO₃H |
| 47 | 5 | NH-COCH₃ | SO₃H |
| 48 | 4 | H | SO₂-C₂H₄OSO₃H |
| 49 | 4 | SO₃H | SO₂-C₂H₄OSO₃H |
| 50 | 5 | SO₃H | SO₂-C₂H₄OSO₃H |
| 51 | 4 | Cl | SO₃H |
| 52 | 5 | SO₃H | Cl |
| 53 | 4 | NH-COC₆H₅ | SO₃H |
| 54 | 4 | NH-CONH₂ | SO₃H |
| 55 | 4 | NH-CONHC₂H₄SO₃H | SO₃H |

### Beispiel 56

19,0 g des Hydrazons der Formel 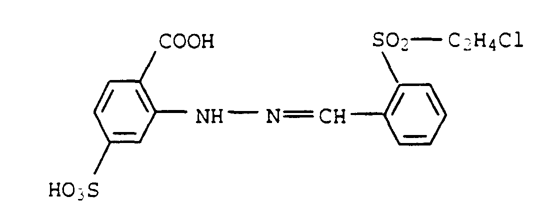 sowie 11,3 g Kupfersulfat-Pentahydrat wurden in 100 ml Wasser suspendiert und durch Zugabe von Natriumcarbonat bei einem pH-Wert von 6,5 bis 7,0 gelöst. Anschließend wurde die wäßrige Diazoniumsalzlösung, die durch übliche wäßrige Diazotierung von 19,7 g 2-Aminophenol-4,6-disulfonsäure erhalten wurde, zugesetzt. Dann wurde weiter verfahren wie in Beispiel 1 beschrieben.

Nach dem Trocknen erhielt man 48 g des kochsalzhaltigen Farbstoffs der Formel

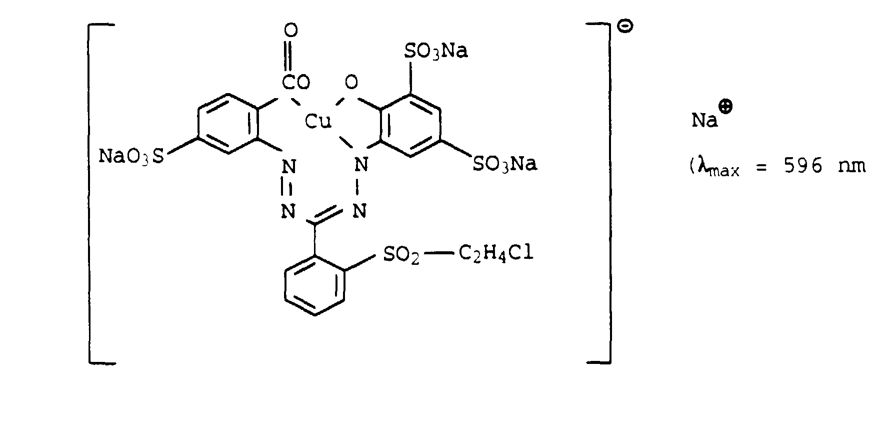

In analoger Weise werden die in der Tabelle 5 aufgeführten Farbstoffe der Formel 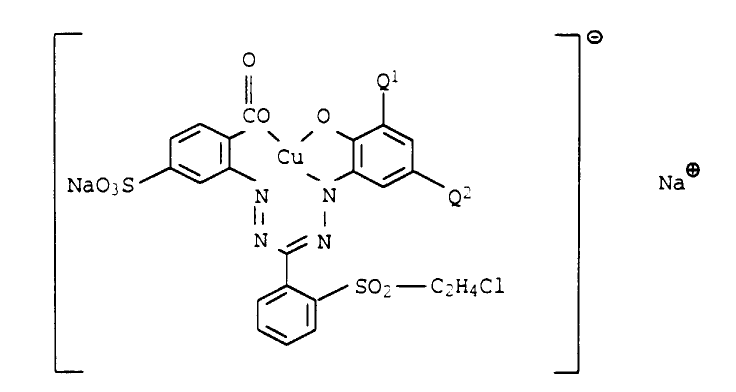 erhalten.

**Tabelle 5**

| Bsp.-Nr. | Q¹ | Q² |
|---|---|---|
| 57 | NH-COCH₃ | SO₃H |
| 58 | SO₃H | SO₂-C₂H₄OSO₃H |
| 59 | Cl | SO₃H |
| 60 | NH-COOC₂H₅ | SO₃H |
| 61 | NH-CONH₂ | SO₃H |
| 62 | COOH | SO₃H |

### Beispiel 63

67,7 g des Hydrazons der Formel 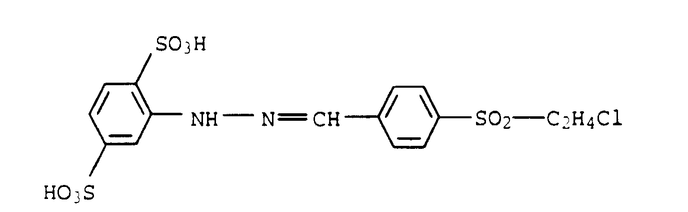 wurden in Gegenwart von 34 g Kupfersulfat-Pentahydrat mit einer wäßrigen Diazoniumsalzlösung, die durch übliche waßrige Diazotierung von 35 g 2-Aminophenol-4,6-disulfonsaure erhalten wurde, analog Beispiel 1 umgesetzt.

Nach dem Trocknen erhielt man 164 g des kochsalzhaltigen Farbstoffs der Formel 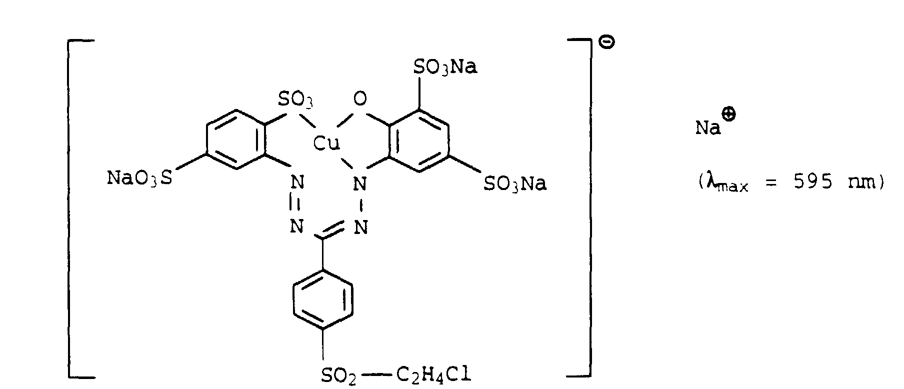 In analoger Weise werden die in der folgenden Tabelle 6 aufgeführten Farbstoffe der Formel 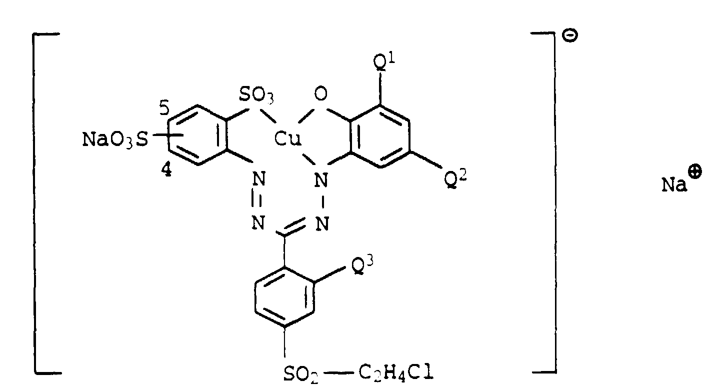 erhalten.

**Tabelle 6**

| Bsp.-Nr. | Position von -SO₃Na | Q¹ | Q² | Q³ |
|---|---|---|---|---|
| 64 | 4 | NH-COCH₃ | SO₃H | H |
| 65 | 5 | SO₃H | SO₃H | Cl |
| 66 | 5 | Cl | SO₃H | H |
| 67 | 5 | SO₃H | SO₂-C₂H₄OSO₃H | H |
| 68 | 5 | H | SO₂-C₂H₄OSO₃H | H |
| 69 | 5 | SO₃H | SO₂-C₂H₄OSO₃H | Cl |
| 70 | 5 | H | SO₂-C₂H₄OSO₃H | Cl |
| 71 | 5 | NH-CONH₂ | SO₃H | H |
| 72 | 4 | NH-CONHC₂H₄OSO₃H | SO₃H | Cl |

### Beispiel 73

58,05 g des Hydrazons der Formel 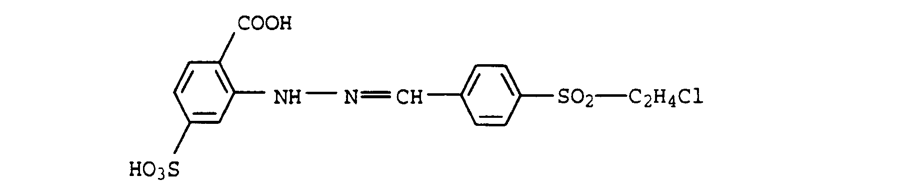 sowie 34,0 g Kupfersulfat-Pentahydrat wurden in 320 ml Wasser suspendiert und durch Zugabe von Natriumcarbonat bei einem pH-Wert von 6,5 bis 7,0 gelöst. Zu dieser Mischung ließ man die Lösung von 87,6 g Diazoxidsäure zulaufen, wobei die Temperatur zwischen 10 bis 20°C und der pH-Wert mit Natriumcarbonat zwischen 6,0 bis 6,5 gehalten wurde.

Es wurde 5 Stunden bei Raumtemperatur nachgerührt, dann die gebildete Kupferkomplexformazan-Verbindung mit Natriumchlorid ausgefällt, abfiltriert, mit verdünnter wäßriger Natriumchloridlösung gewaschen und bei 50°C unter vermindertem Druck getrocknet.

Man erhielt 231 g des kochsalzhaltigen Farbstoffs 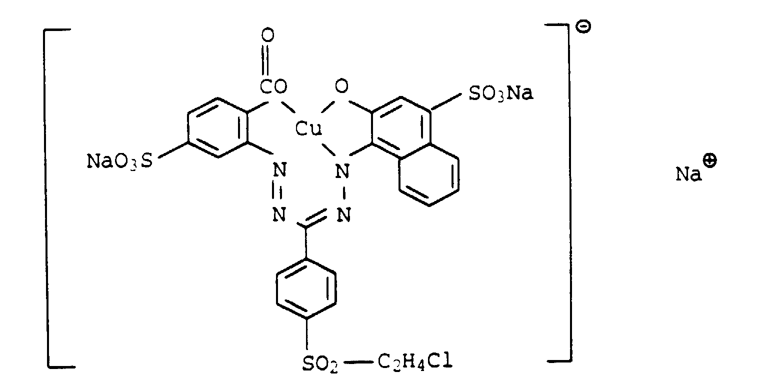 Er liefert auf Baumwolle grüne Färbungen mit guter Naßechtheit und guter Lichtechtheit.

In analoger Weise werden die in der folgenden Tabelle 7 aufgeführten Farbstoffe erhalten. (Die in Beispiel 76 bis 79 erhaltenen Kupferkomplexfarbstoffe wurden durch Nachrühren bei einem pH-Wert von 1 analog Beispiel 30 in die jeweils stabilere Komplexform übergeführt.)

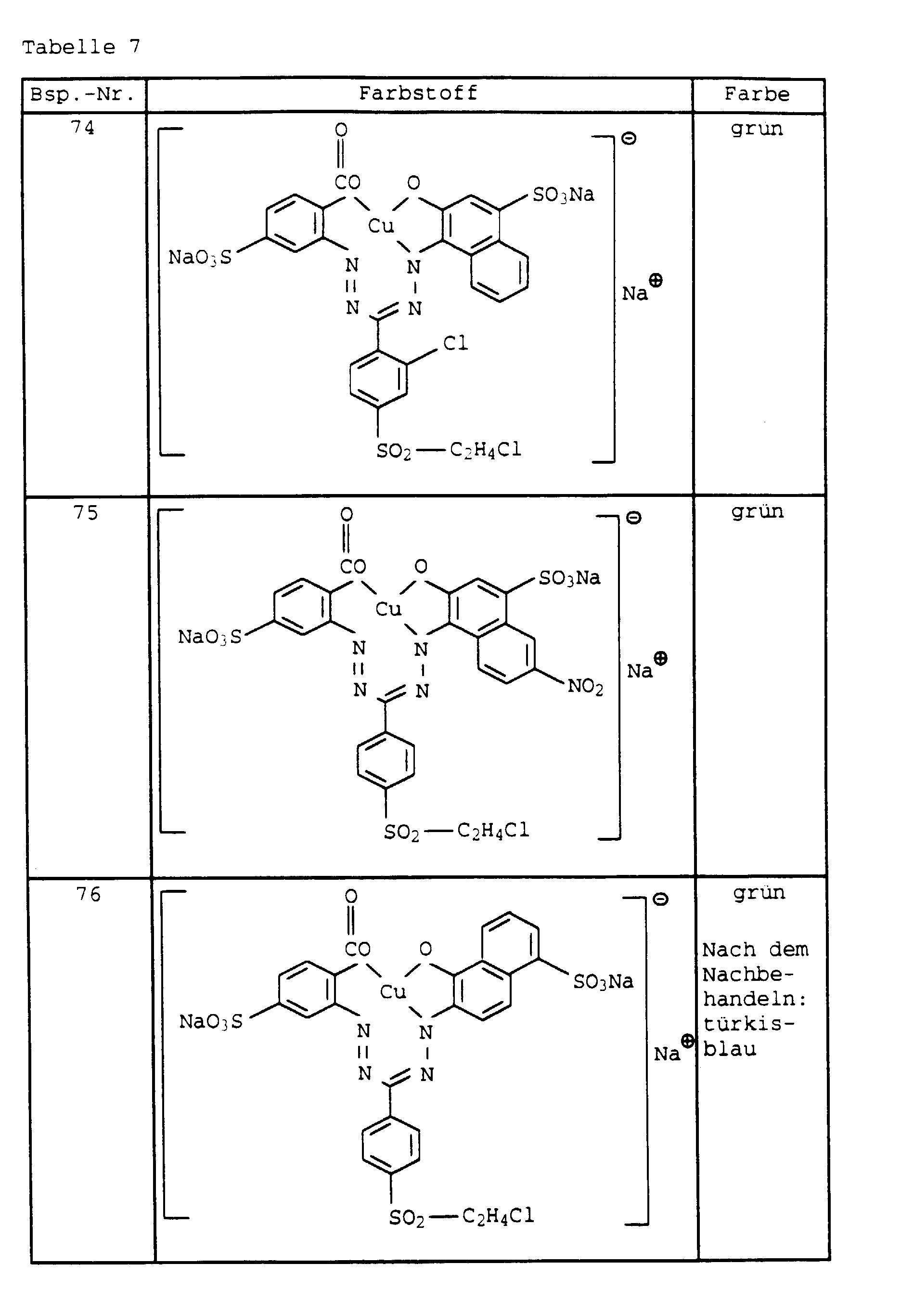

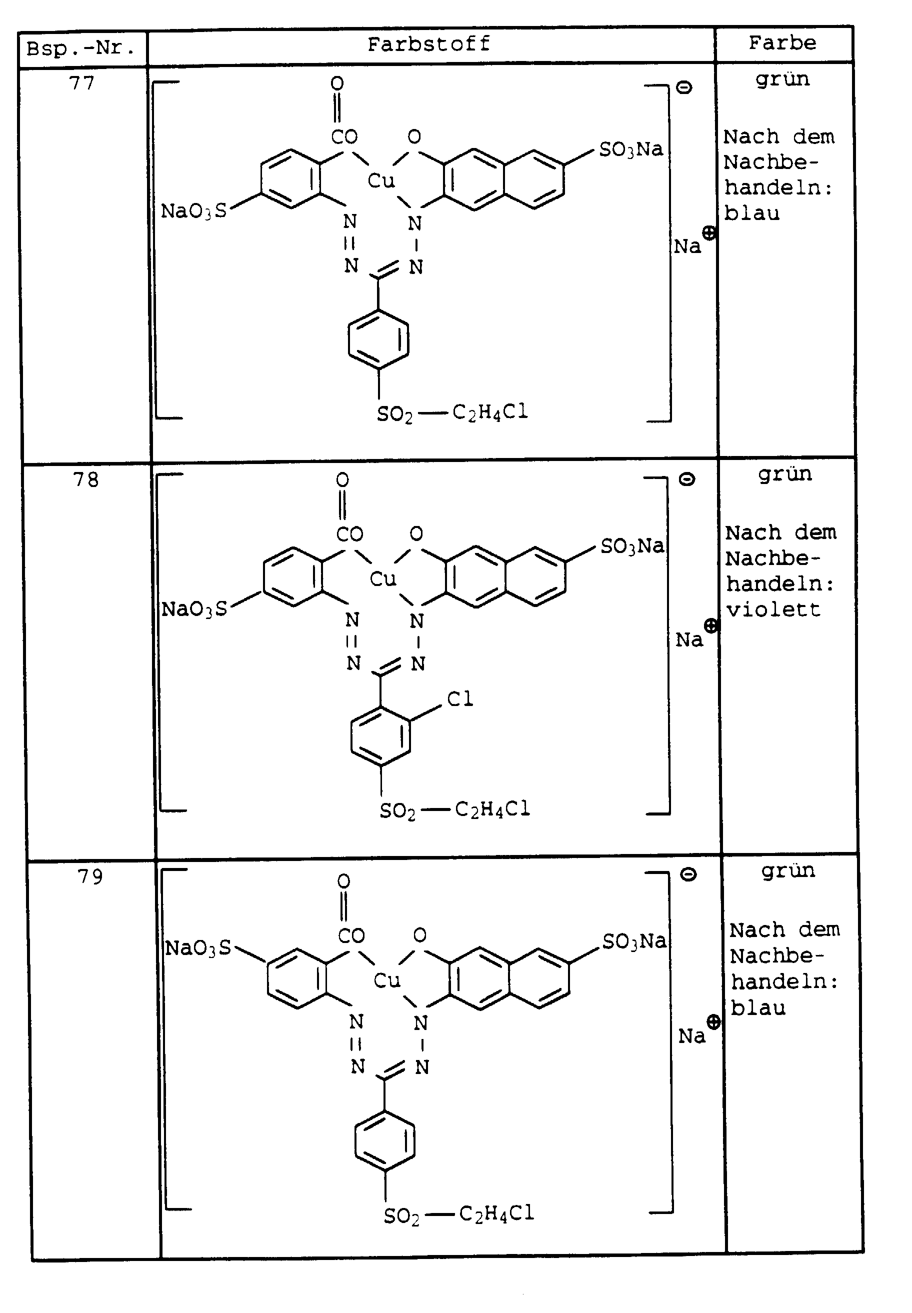

### Beispiel 80

66,9 g 2-Carboxyphenylhydrazin-5-sulfonsaure wurden in 300 ml Wasser durch Zugabe von 10 gew.-%iger Natronlauge bei einem pH-Wert von 6 gelöst. Es wurden 66,9 g 4-(2-Chlorethylsulfonyl)benzaldehyd zugegeben und mit 7,5 ml konzentrierter Salzsäure ein pH-Wert von 1 eingestellt. Anschließend wurde auf 85 bis 90°C erwärmt und ca. 4 Stunden bei dieser Temperatur nachgerührt, bis die Reaktionskontrolle mittels DC vollständige Kondensation anzeigte, dabei wurde durch Zugabe von 10 gew.-%iger Salzsäure der pH-Wert zwischen 1,0 und 1,3 gehalten. Nach dem Abkühlen wurde das gebildete Hydrazon abgesaugt, mit Wasser neutral gewaschen und bei 40°C unter vermindertem Druck getrocknet. Man erhielt 119 g Hydrazons der Formel 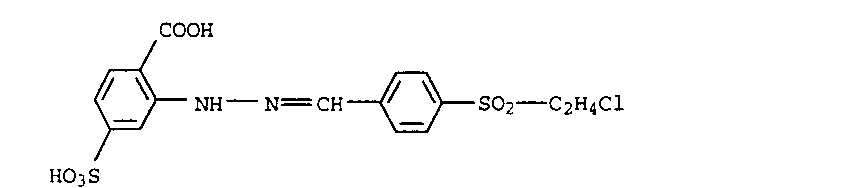 (Reinheit > 98 % - HPLC) Cl_{gef} = 7,51 Cl_{ber} = 7,93
- ¹H-NMR (DMSO): δ =: 3,90 (CH₂CH₂), 7,30, 7,95, 8,00, 8,20, 8,30 (Aromaten-H, CH=N), 11,5 (NH) ppm
- ¹³C-NMR (DMSO): δ =: 36,4, 56,9, 110,8, 111,0, 116,0, 126,7, 128,3, 131,2, 138,1, 139,0, 140,5, 146,2, 152,8, 169,0 ppm

Analog Beispiel 80 werden die in der folgenden Tabelle 8 aufgeführten Hydrazone erhalten.

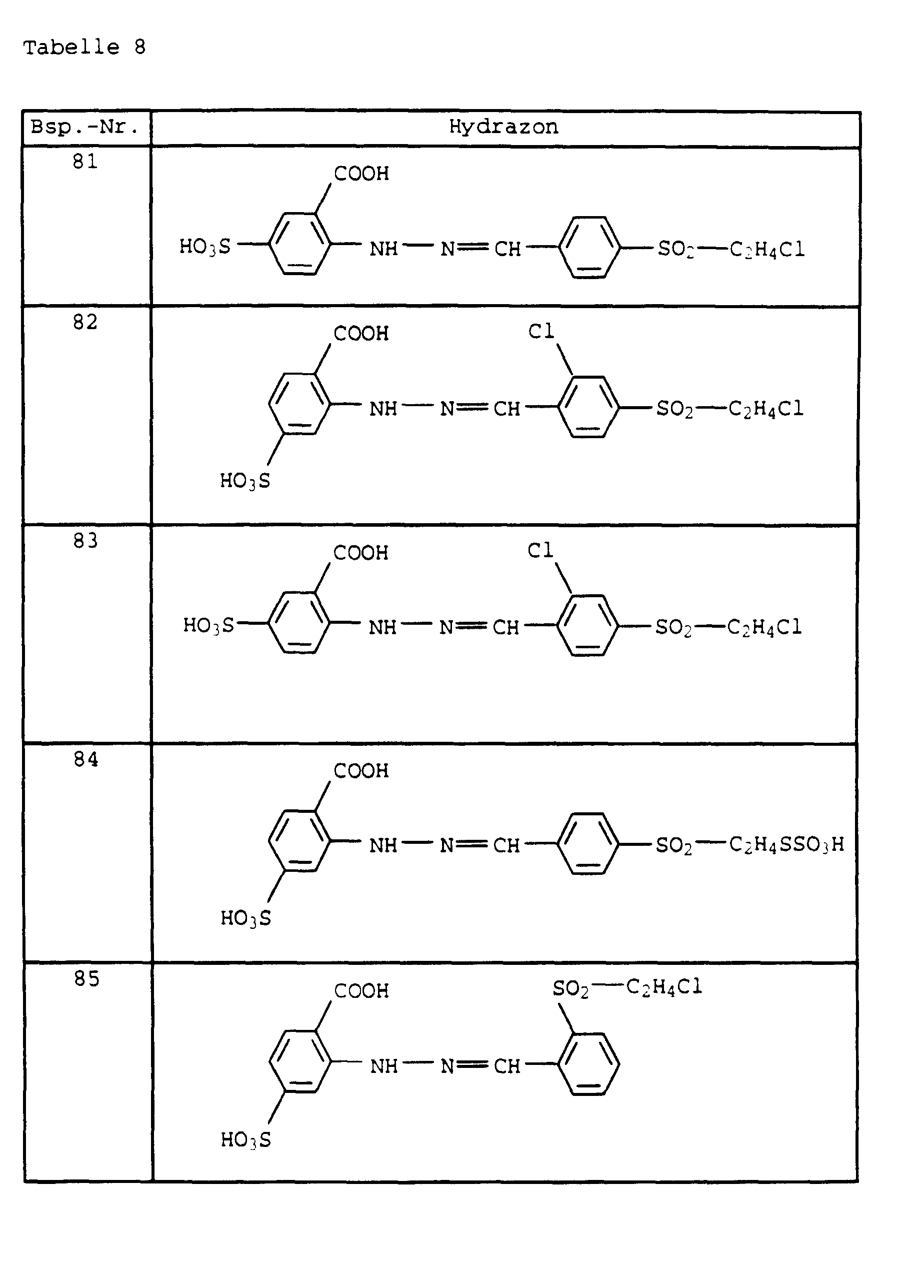

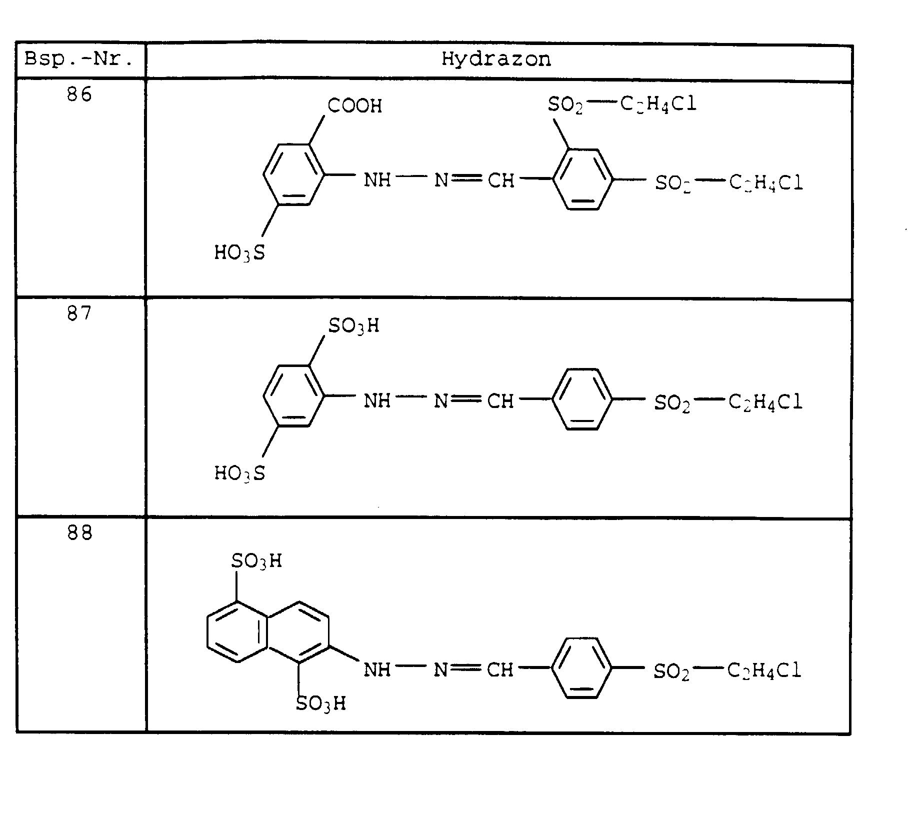

## Patentansprüche

1. Formazanfarbstoffe der Formel I 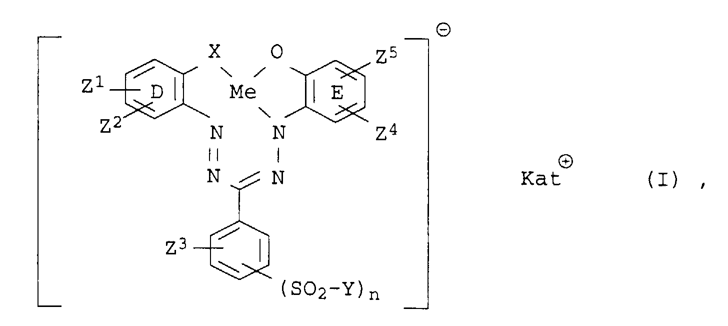 in der
n 1 oder 2,
Kat^{⊕} das Äquivalent eines Kations,
Me Kupfer oder Nickel,
X einen Rest der Formel CO-O oder SO₂-O
Y Vinyl oder einen Rest der Formel C₂H₄-Q, worin Q für eine unter alkalischen Reaktionsbedingungen abspaltbare Gruppe steht,
Z¹ Wasserstoff oder Hydroxysulfonyl,
Z² Wasserstoff, C₁-C₄-Alkanoylamino, Halogen oder Nitro,
Z³ Wasserstoff, Halogen, C₁-C₄-Alkyl, C₁-C₄-Alkoxy, C₁-C₄-Alkylsulfonyl, Nitro oder Hydroxysulfonyl und
Z⁴ und Z⁵ unabhängig voneinander jeweils Hydroxysulfonyl, Halogen, Nitro, Carboxyl, Ureido, gegebenenfalls substituiertes C₁-C₄-Mono- oder Di-alkylureido, gegebenenfalls substituiertes Phenylureido, gegebenenfalls substituiertes Phenyl, gegebenenfalls substituiertes C₁-C₄-Alkanoylamino, gegebenenfalls substituiertes Benzoylamino, C₁-C₄-Alkoxycarbonylamino, gegebenenfalls substituiertes Mono- oder Dialkylcarbamoyl, gegebenenfalls substituiertes Mono- oder Dialkylsulfamoyl, einen Rest der Formel SO₂-Y oder einer der beiden Reste Z⁴ und Z⁵ auch Wasserstoff bedeuten, und
die Ringe D und E benzoanelliert sein können.

2. Formazanfarbstoffe nach Anspruch 1, dadurch gekennzeichnet, daß Me Kupfer bedeutet.

3. Formazanfarbstoffe nach Anspruch 1, dadurch gekennzeichnet, daß n 1 bedeutet.

4. Formazanfarbstoffe nach Anspruch 1, dadurch gekennzeichnet, daß Y Vinyl oder einen Rest der Formel C₂H₄-Q bedeutet, worin Q für Chlor, Sulfato oder Thiosulfato steht.

5. Hydrazone der Formel II 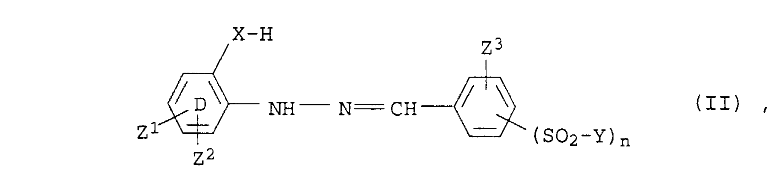 in der
n 1 oder 2,
Y Vinyl oder einen Rest der Formel C₂H₄-Q, worin Q für eine unter alkalischen Reaktionsbedingungen abspaltbare Gruppe steht,
X einen Rest der Formel CO-O oder SO₂-O,
Z¹ Wasserstoff oder Hydroxysulfonyl,
Z² Wasserstoff, C₁-C₄-Alkanoylamino, Halogen oder Nitro und
Z³ Wasserstoff, Halogen, C₁-C₄-Alkyl, C₁-C₄-Alkoxy, C₁-C₄-Alkylsulfonyl, Nitro oder Hydroxysulfonyl bedeuten und
der Ring D benzoanelliert sein kann.

6. Verwendung der Formazanfarbstoffe gemäß Anspruch 1 zum Färben oder Bedrucken von Hydroxygruppen oder Stickstoffatome aufweisenden organischen Substraten.

## Claims

1. Formazan dyes of the formula I 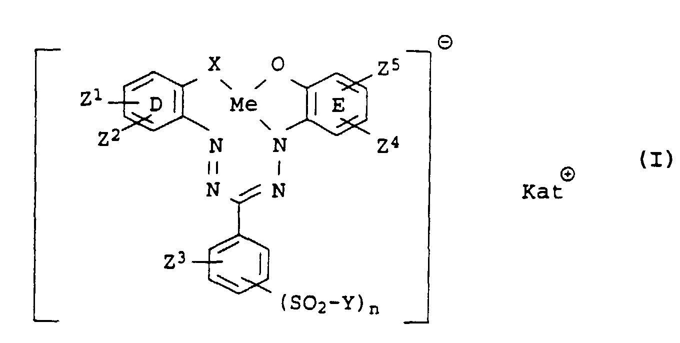 where
n is 1 or 2,
Kat^{⊕} is the equivalent of a cation,
Me is copper or nickel,
X is a radical of the formula CO-O or SO₂-O,
Y is vinyl or a radical of the formula C₂H₄-Q, where Q is a group which is detachable under alkaline reaction conditions,
Z¹ is hydrogen or hydroxysulfonyl,
Z² is hydrogen, C₁-C₄-alkanoylamino, halogen or nitro,
Z³ is hydrogen, halogen, C₁-C₄-alkyl, C₁-C₄-alkoxy, C₁-C₄-alkylsulfonyl, nitro or hydroxysulfonyl,
Z⁴ and Z⁵ are independently of one another hydroxysulfonyl, halogen, nitro, carboxyl, ureido, substituted or unsubstituted mono- or di(C₁-C₄-alkyl)-ureido, substituted or unsubstituted phenylureido, substituted or unsubstituted phenyl, substituted or unsubstituted C₁-C₄-alkanoylamino, substituted or unsubstituted benzoylamino, C₁-C₄-alkoxycarbonylamino, substituted or unsubstituted mono- or dialkylcarbamoyl, substituted or unsubstituted mono-or dialkylsulfamoyl, a radical of the formula SO₂-Y or one of the two radicals Z⁴ and Z⁵ is also hydrogen, and
the rings D and E may be benzofused.

2. Formazan dyes as claimed in claim 1, wherein Me is copper.

3. Formazan dyes as claimed in claim 1, wherein n is 1.

4. Formazan dyes as claimed in claim 1, wherein Y is vinyl or a radical of the formula C₂H₄-Q, where Q is chlorine, sulfato or thiosulfato.

5. Hydrazones of the formula II 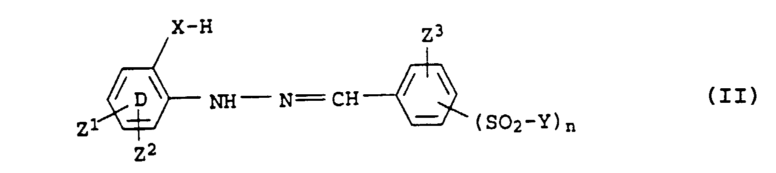 where
n is 1 or 2,
Y is vinyl or a radical of the formula C₂H₄-Q, where Q is a group which is detachable under alkaline reaction conditions,
X is a radical of the formula CO-O or SO₂-O,
Z¹ is hydrogen or hydroxysulfonyl,
Z² is hydrogen, C₁-C₄-alkanoylamino, halogen or nitro,
Z³ is hydrogen, halogen, C₁-C₄-alkyl, C₁-C₄-alkoxy, C₁-C₄-alkylsulfonyl, nitro or hydroxysulfonyl, and
the ring D may be benzofused.

6. The use of the formazan dyes of claim 1 for dyeing or printing hydroxyl- or nitrogen-containing organic substrates.

## Revendications

1. Colorants de formazane répondant à la Formule I 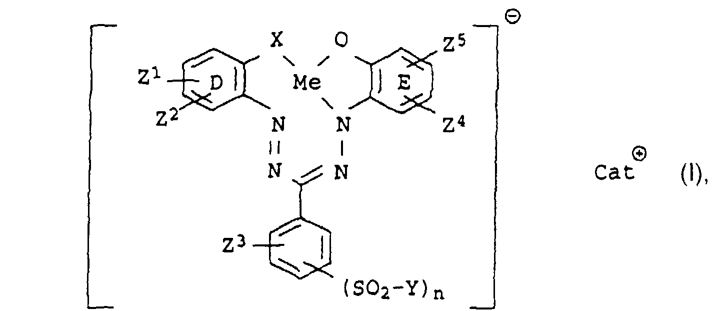 dans laquelle
n vaut 1 ou 2,
Cat^{⊕} représente l'équivalent d'un cation,
Me représente un atome de cuivre ou un atome de nickel,
X représente un résidu répondant à la formule CO-O ou SO₂-O,
Y représente un groupe vinyle ou un résidu répondant à la formule C₂H₄-Q, où Q représente un groupe pouvant être clivé dans des conditions de réaction alcalines,
Z¹ représente un atome d'hydrogène ou un groupe hydroxysulfonyle,
Z² représente un atome d'hydrogène, un groupe (alcanoyle en C₁-C₄)amino, un atome d'halogène ou un groupe nitro,
Z³ représente un atome d'hydrogène, un atome d'halogène, un groupe alkyle en C₁-C₄, un groupe alcoxy en C₁-C₄, un groupe (alkyle en C₁-C₄)sulfonyle, un groupe nitro ou un groupe hydroxysulfonyle et
Z⁴ et Z⁵ représentent indépendamment l'un de l'autre respectivement un groupe hydroxysulfonyle, un atome d'halogène, un groupe nitro, un groupe carboxyle, un groupe uréido, un groupe mono(alkyle en C₁-C₄)- ou di(alkyle en C₁-C₄)uréido éventuellement substitué, un groupe phényluréido éventuellement substitué, un groupe phényle éventuellement substitué, un groupe (alcanoyle en C₁-C₄)amino éventuellement substitué, un groupe benzyolamino éventuellement substitué, un groupe (alcoxy en C₁-C₄)carbonylamino, un groupe mono-ou dialkylcarbamoyle éventuellement substitué, un groupe mono- ou dialkylsulfamoyle éventuellement substitué, un résidu répondant à la formule SO₂-Y ou à un des deux résidus Z⁴ et Z⁵ pouvant également représenter un atome d'hydrogène, et
les noyaux D et E pouvant être benzocondensés.

2. Colorants de formazane selon la revendication 1, caractérisés en ce que Me signifie un atome de cuivre.

3. Colorants de formazane selon la revendication 1, caractérisés en ce que n vaut 1.

4. Colorants de formazane selon la revendication 1, caractérisés en ce que Y représente un groupe vinyle ou un résidu répondant à la formule C₂H₄-Q, où Q représente un atome de chlore, un groupe sulfato ou un groupe thiosulfato.

5. Hydrazones répondant à la Formule II 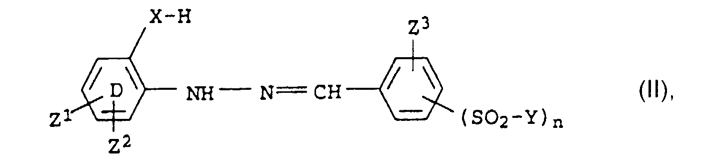 dans laquelle
n vaut 1 ou 2,
Y représente un groupe vinyle ou un résidu répondant à la formule C₂H₄-Q, où Q représente un groupe pouvant être clivé dans des conditions de réaction alcalines,
X représente un résidu répondant à la formule CO-O ou SO₂-O,
Z¹ représente un atome d'hydrogène ou un groupe hydroxysulfonyle,
Z² représente un atome d'hydrogène, un groupe (alcanoyle en C₁-C₄)amino, un atome d'halogène ou un groupe nitro,
et
Z³ représente un atome d'hydrogène, un atome d'halogène, un groupe alkyle en C₁-C₄, un groupe alcoxy en C₁-C₄, un groupe (alkyle en C₁-C₄)sulfonyle, un groupe nitro ou un groupe hydroxysulfonyle et
le noyau D peut être benzocondensé.

6. Utilisation des colorants de formazane selon la revendication 1, pour la coloration et l'impression de substrats organiques présentant des groupes hydroxyles ou des atomes d'azote.
